# EUROPÄISCHE PATENTSCHRIFT

(11) **EP 2 946 739 B1**
(45) Veröffentlichungstag und Bekanntmachung des Hinweises auf die Patenterteilung: **12.10.2016**
(21) Anmeldenummer: 15168389.3
(22) Anmeldetag: 20.05.2015
(51) Int. Cl.: A61B 18/14, A61B 18/00, A61B 17/00

(54) **SONDENAPPLIKATOR**
PROBE APPLICATOR
APPLICATEUR DE SONDE

(30) Priorität: 20.05.2014 DE 102014107087
(43) Veröffentlichungstag der Anmeldung: 25.11.2015
(73) Patentinhaber: BOWA-electronic GmbH & Co. KG, 72810 Gomaringen (DE)
(72) Erfinder: Kleih, Jörg, 72810 Gomaringen (DE); Doppelstein, Alexander, 78549 Spaichingen (DE); Hluchy, Heinz, 72116 Mössingen (DE)
(74) Vertreter: Schneider, Peter Christian

(56) Entgegenhaltungen:
- EP-B1- 1 607 061
- CN-U- 202 397 597
- US-A- 5 472 441
- US-A1- 2005 107 779
- US-B1- 6 231 571

## Beschreibung

### Gebiet der Erfindung

Die Erfindung betrifft einen Sondenapplikator zur Positionierung einer bipolaren elektrochirurgischen Sonde mit einem abwinkelbaren distalen Sondenende, das eine Sondenspitze mit einem bipolaren Elektrodenkopf aufweist, der mit zwei in einer flexiblen Sondenhülle geführten elektrischen Leitern verbunden ist, einem Schaftrohr, in dem die Sonde geführt wird, und einem Handstück, über das das Sondenende selektiv abbiegbar ist.

### Stand der Technik

Sondenapplikatoren werden beispielsweise für die Minimalinvasive Chirurgie (MIS) von Bandscheibenvorfällen als bipolare Vaporisationssonden (zur perkutanen Nukleustherapie) eingesetzt.

Aus der EP 1 607 061 B1 ist ein Sondenapplikator zur Positionierung einer bipolaren elektrochirurgischen Sonde mit einem abwinkelbaren distalen Sondenende bekannt. Das distale Sondenende weist eine Sondenspitze mit einem bipolaren Elektrodenkopf auf, der mit zwei in einer Sondenhülle geführten elektrischen Leitern verbunden ist. Die Sonde wird über ein Handstück in einem Schaft längsverschieblich geführt. Das Sondenende ist über das Handstück abbiegbar. Dabei ist das distale Ende der Sondenhülle vorgebogen und wird bei Abdeckung durch das distale Schaftrohrende in eine gestreckte Stellung gespannt.

Nachteilig bei dem bekannten Sondenapplikator ist, dass die Sondenhülle selbst eine vorgebogene Form bzw. Spannung aufweist. Weiterhin nachteilig ist, dass bei zwei parallel zueinander geführten Leitern besonders bei starker Krümmung relativ hohe Scherkräfte auf den außen liegenden Leiter (Elektrode) wirken. Sobald die Sondenspitze in ihre gestreckte gerade, das heißt gegenüber deren Längsachse nicht ausgelenkte Stellung gebracht wird, führt dies bei der außen liegenden Elektrode zu einer Stauchung, die die außen liegenden Elektrode belastet. Zudem wird die relative Bewegung zwischen Sonde und außen liegendem Schaft wegen erhöhter Reibkräfte behindert.

Weiterhin ist aus der DE 600 28 863 T2 ein ähnlicher Sondenapplikator bekannt, bei dem die Sondenhülle eine vorgespannte gebogene Form aufweist. Die Sondenhülle soll dabei aus einem Material gefertigt sein, das vorgebogen werden kann und einen ausreichenden Speicher aufweist, um seine vorgebogene Form beizubehalten, wenn die Sondenhülle bzw. die Sonde aus dem entgegenwirkenden äußeren Rohr nach außen verlängert wird.

Auch dieser Sondenapplikator weist die oben beschriebenen Nachteile auf.

### Aufgabenstellung

Die Aufgabe der vorliegenden Erfindung ist es, die Gestaltung der vorgespannten Abbiegung der Sondenspitze zu vereinfachen und die Belastung insbesondere der außen liegenden Elektroden bei einer Stauchung zu verringern.

### Darlegung der Erfindung

Diese Aufgabe wird in Verbindung mit den Merkmalen des Oberbegriffs von Anspruch 1 dadurch gelöst, dass der erste elektrische Leiter im Bereich des distalen Sondenendes eine gerichtete gekrümmte Vorspannung aufweist, durch die bei zurückgezogenem Schaftrohrende das freiliegende Sondenende der flexiblen Sonde in seine abgewinkelte, vorgebogene Stellung gedrückt wird und bei ausgefahrenem Schaftrohr das Sondenende von dem umgebenden Schaftrohrende in seine gestreckte Stellung gespannt wird, und dass der zweite elektrische Leiter als ein flexibles Drahtseil ausgebildet ist.

Dadurch, dass die gekrümmte Vorspannung des distalen Sondenendes nicht über die Sondenhülle sondern über die erste Elektrode erfolgt, wird die Vorspannung erheblich vereinfacht. Durch die Ausbildung der außen liegenden Elektrode als flexibles Drahtseil wird die beim Geradestrecken notwendige Stauchung der Elektrode bei geringerer Belastung erheblich verbessert.

Gemäß einer bevorzugten Ausführungsform der Erfindung ist der erste elektrische Leiter zur Abwicklungsrichtung hin innen und der zweite elektrische Leiter der Abwicklungsrichtung weg außen angeordnet. Der erste elektrische Leister ist im Bereich der gekrümmten Vorspannung des distalen Sondenendes flach mit zwei einander gegenüberliegenden Seitenflächen ausgebildet. Der elektrische Leiter kann dabei zumindest in diesem Bereich aus einem durch Vergüten behandelnden Teil wie Edelstahl bestehen, beispielsweise durch Walzen in eine vorgebogene Form gebracht werden. Die Sondenhülle dabei aus einem flexiblen Kunststoff ausgebildet sein.

Nach einer weiteren bevorzugten Ausführungsform der Erfindung ist das flexible Drahtseil aus einer Mehrzahl von Litzen von jeweils einer Mehrzahl von Drähten ausgebildet. Ein solches Drahtseil lässt sich besonders leicht stauchen, da es dabei auffächern kann. Beispielsweise kann das Drahtseil aus einer ungeraden Zahl von Litzen mit jeweils einer ungeraden Zahl von Drähten ausgebildet sein. Dabei kann sich jeweils ein Kern oder eine Seele bilden um den eine Mehrzahl von Drähten oder Litzen herum angeordnet ist.

Gemäß einer weiteren bevorzugten Ausführungsform der Erfindung ist die flexible Sondenhülle im Querschnitt ringförmig ausgebildet und mindestens einer der elektrischen Leiter weist eine isolierende Hülle auf. Dies hat zum einen den Vorteil, dass die Sondenhülle als einfacher Schlauch ausgebildet sein kann und die beiden in der Hülle parallel nebeneinander geführten elektrischen Leiter gegeneinander isoliert sind.

Grundsätzlich ist es aber auch möglich, die flexible Sondenhülle im Querschnitt innen eine sich mittig verengende Kontur zu versehen, in der die elektrischen Leiter beabstandet zueinander geführt werden. Dies führt zu einer Isolierung durch Luft, wobei es nicht notwendig ist, dass einer der beiden Leiter isoliert ist.

Nach einer weiteren bevorzugten Ausführungsform der Erfindung weist die flexible Sondenhülle eine Trennwand auf, die das Lumen der Sondenhülle in zwei voneinander getrennte Kammern unterteilt, in denen die elektrischen Leiter geführt werden. Auch hierbei eine Isolierung der elektrischen Leiter selbst nicht notwendig. Entsprechend ist die Sondenhülle aus einem elektrisch isolierenden Kunststoff ausgebildet.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine Seitenansicht eines Sondenapplikators mit ausgefahrener und abgewinkelter Sondenspitze,
- Figur 2:: eine vergrößerte Darstellung der Sondenspitze mit distalem Schaftrohrende von Fig. 1 im Schnitt,
- Figur 3:: eine vergrößerte Darstellung der Sondenspitze von Fig. 2 entlang der Linie III-III geschnitten,
- Figur 4:: eine vergrößerte Darstellung der Sondenspitze mit distalem Schaftrohr in gestreckter Stellung bei von dem Schaftrohrende abgedecktem distalen Ende der Sondenspitze,
- Figur 5:: eine Sondenspitze eines weiteren Sondenapplikators in vergrößerter Darstellung im Schnitt entsprechend Fig. 2,
- Figur 6:: eine vergrößerte Darstellung der Sondenspitze von Fig. 5 im Querschnitt entsprechend der Darstellung von Fig. 3,
- Figur 7:: eine vergrößerte Darstellung einer weiteren Sondenspitze im Querschnitt entsprechend der Darstellung von Fig. 3 und
- Figur 8:: eine vergrößerte Darstellung einer weiteren Sondenspitze im Querschnitt entsprechend der Darstellung von Fig. 3.

### Beschreibung bevorzugter Ausführungsformen

Ein Sondenapplikator 1 besteht im Wesentlichen aus einer Sonde 2, einem Schaftrohr 3 und einem Handstück 4.

Die Sonde 2 ist als eine bipolare elektrochirurgische Sonde mit einem abwinkelbaren distalen Sondenende 5 ausgebildet. Die Sonde weist eine flexible Sondenhülle 6 aus einem Kunststoff auf. In der Sondenhülle 6 werden ein erster elektrischer Leiter 7 und ein zweiter elektrischer Leiter 8, die parallel und beabstandet zueinander angeordnet sind, geführt. Am distalen Sondenende 5 ist ein bipolarer Elektrodenkopf 9 mit einem ersten Pol 10 und einem zweiten Pol 11 angeordnet, der die Sondenhülle 6 zentral abschließt und mit dessen Polen 10, 11 die elektrischen Leiter 7, 8 verbunden sind.

Der erste elektrische Leiter 8 weist im Bereich des distalen Sondenendes 5 eine gerichtete Vorspannung auf, die das freiliegende distale Sondenende 5 in seine vorgebogene, abgewinkelte Stellung drückt. Der erste elektrische Leiter 7 ist im Bereich der gekrümmten Vorspannung des distalen Sondenendes 5 flach mit zwei einander gegenüberliegenden Seitenflächen 12, 13 ausgebildet. Die notwendige Vorspannung kann dabei beispielsweise durch Walzen erzeugt werden.

Der zweite elektrische Leiter 8 ist als ein flexibles Drahtseil ausgebildet und besteht aus einer Mehrzahl von Litzen 14. Jede Litze 14 besteht dabei aus einer Mehrzahl von Drähten 15.

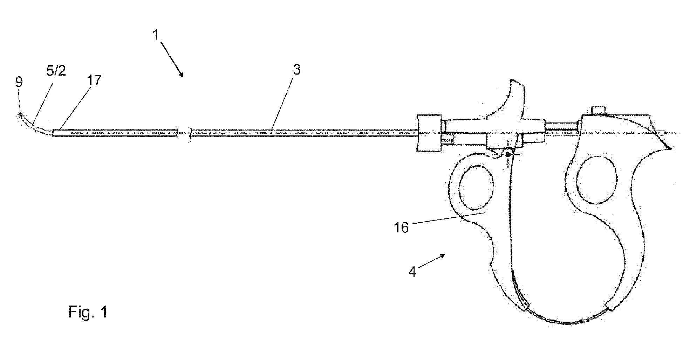

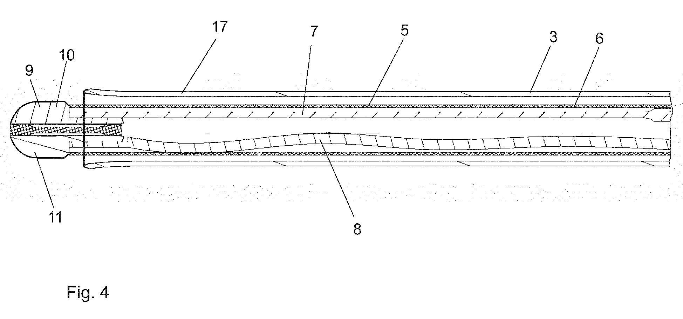

Die Sonde 2 wird mit ihrer Sondenhülle 6 in dem Schaftrohr 3 längsverschieblich geführt. Die Sonde 2 und das Schaftrohr 3 sind jeweils mit dem Handstück 4 verbunden über dessen Griff 16 die Sonde 2 und das Schaftrohr 3 in ihrer Längsrichtung relativ gegeneinander verschoben werden können. Gibt das distale Schaftrohrende 17 das distale Sondenende 5 frei, befindet sich das distale Sondenende 5 in seiner von der Vorspannung maximal ausgelenkten Abwinklungsstellung (s. Fig. 1, 2 und 5). Wird das distale Sondenende 5 von dem distalen Schaftrohrende 17 abgedeckt, befindet sich das distale Sondenende 5 in seiner nicht ausgelenkten, gestreckten Stellung (s. Fig. 4).

Der Sonde 2 weist innen zur Abwinklungsrichtung hin den ersten elektrischen Leiter 7 auf, der damit um den kleineren Innenradius 18 gebogen wird. Von der Abwinklungsrichtung weg weist die Sonde 2 außen in ihrem Lumen den zweiten elektrischen Leiter 8 auf, der damit um den größeren Innenradius 19 gebogen wird.

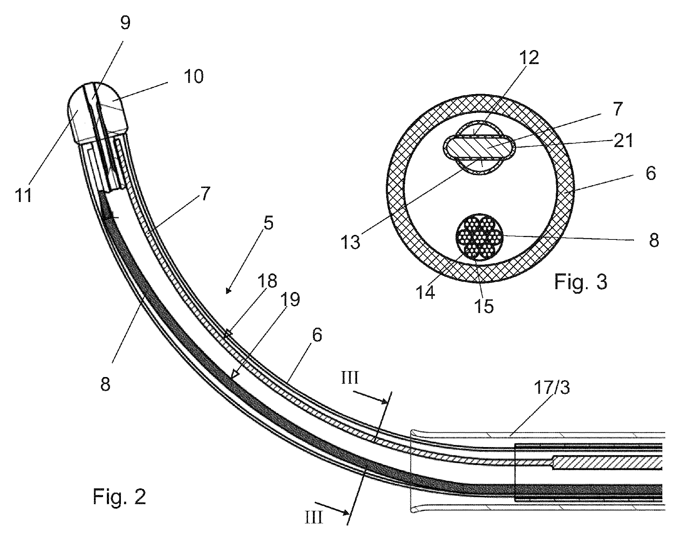

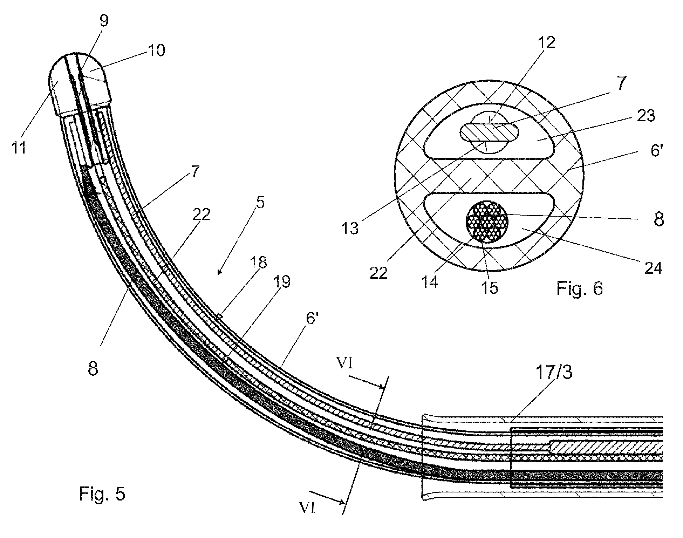

Entsprechend dem Ausführungsbeispiel der Figuren 2 und 3 ist die flexible Sondenhülle 6 im Querschnitt ringförmig ausgebildet, das heißt, die Sondenhülle 6 weist eine ringförmige Wandung 20 auf, wobei der erste Elektrische Leiter 7 mit einer Isolierung 21 versehen ist. Entsprechend dem Ausführungsbeispiel der Figuren 5 und 6 ist die flexible Sondenhülle 6' im Querschnitt ringförmig mit einer mittig angeordneten Trennwand 22 versehen, die das Lumen der Sondenhülle 6' in zwei Kammern 23, 24 unterteilt, die jeweils einen elektrischen Leiter 7, 8 aufnehmen, so dass keiner der elektrischen Leiter 7, 8 eine eigene Isolierung benötigt.

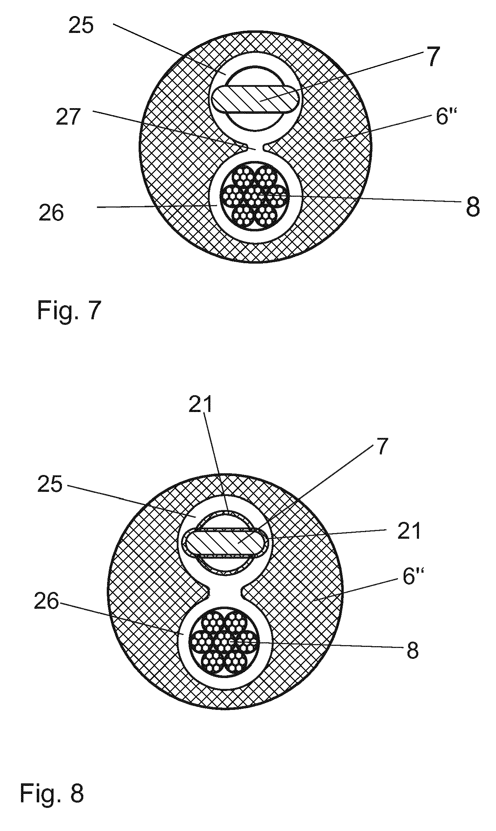

Entsprechend dem Ausführungsbeispiel der Figur 7 weist das Lumen der Sondenhülle 6" zwei ineinander übergehende, miteinander verbundene Kanäle 25, 26 auf, die die elektrischen Leiter 7, 8 durch einen Luftzwischenraum 27 voneinander isolieren.

Das Ausführungsbeispiel der Figur 8 ist entsprechend Figur 7 ausgebildet, der erste elektrische Leiter 7 weist jedoch eine Isolierung 21 auf.

Der Sondenapplikator 1 wird mit von dem distalen Schaftrohrende 17 abgedeckten distalen Sondenende 5, also mit gestrecktem - nicht abgewinkeltem Sondenende 5 - am Operationsort platziert, wobei durch Betätigung des Griffes 16 das Schaftrohr 3 gegenüber der Sonde 2 zurückgezogen wird, so dass das nicht mehr abgedeckte distale Sondenende 5 in seine abgewinkelte Position gespannt wird.

Natürlich stellen die in der speziellen Beschreibung diskutierten und in den Figuren gezeigten Ausführungsformen nur illustrative Ausführungsbeispiele der vorliegenden Erfindung dar.

### Bezugszeichenliste

- 1: Sondenapplikator
- 2: Sonde
- 3: Schaftrohr
- 4: Handstück
- 5: distales Sondenende
- 6, 6', 6": Sondenhülle
- 7: erster elektrischer Leiter
- 8: zweiter elektrischer Leiter
- 9: Elektrodenkopf
- 10: erster Pol von 9
- 11: zweiter Pol von 9
- 12: erste Seitenfläche von 7
- 13: zweite Seitenfläche von 7
- 14: Litze von 8
- 15: Draht von 14
- 16: Griff von 4
- 17: distales Schaftrohrende
- 18: Innenradius von 7, kleiner
- 19: Innenradius von 8, großer
- 20: ringförmige Wandung von 6
- 21: isolierende Hülle von 7
- 22: Trennwand von 6'
- 23: erste Kammer von 6'
- 24: zweite Kammer von 6'
- 25: Kanal von 6"
- 26: Kanal von 6"
- 27: Luftzwischenraum von 6"

## Patentansprüche

1. Sondenapplikator (1) und bipolare elektrochirurgische Sonde (2) mit einem abwinkelbaren distalen Sondenende (5), das eine Sondenspitze mit einem bipolaren Elektrodenkopf (9) aufweist, der mit zwei in einer Sondenhülle (6, 6', 6") geführten elektrischen Leitern (7, 8) verbunden ist;
wobei der Sondenapplikator zur Positionierung der Sonde ausgeführt ist, mit einem Schaftrohr (3), in dem die Sonde (2) geführt wird, und einem Handstück (4), über das das Sondenende (5) abbiegbar ist, wobei das Schaftrohr (3) starr ausgebildet ist und das Schaftrohr (3) und die Sondenhülle (6, 6', 6") in Längsrichtung relativ zueinander längsverschieblich sind, wobei das distale Sondenende (5) vorgebogen ist und bei Abdeckung durch das distale Schaftrohrende (17) in eine gestreckte Stellung gespannt wird,
**dadurch gekennzeichnet,**
**dass** der erste elektrische Leiter (7) im Bereich des distalen Sondenendes (5) eine gerichtete gekrümmte Vorspannung aufweist, durch die bei zurückgezogenem Schaftrohrende (17) das freiliegende Sondenende (5) der flexiblen Sonde (2) in seine abgewinkelte, vorgebogene Stellung gedrückt wird und bei ausgefahrenem Schaftrohr (3) das Sondenende (5) von dem umgebenden Schaftrohrende (17) in seine gestreckte Stellung gespannt wird, und
**dass** der zweite elektrische Leiter (8) als ein flexibles Drahtseil ausgebildet ist.

2. Sondenapplikator nach Anspruch 1,
**dadurch gekennzeichnet,**
**dass** der erste elektrische Leiter (7) im Bereich der gekrümmten Vorspannung des distalen Sondenendes (5) flach mit zwei einander gegenüberliegenden Seitenflächen (12, 13) ausgebildet ist.

3. Sondenapplikator nach Anspruch 1 oder 2,
**dadurch gekennzeichnet,**
**dass** das flexible Drahtseil aus einer Mehrzahl von Litzen (14) von jeweils einer Mehrzahl von Drähten (15) ausgebildet ist.

4. Sondenapplikator nach Anspruch 3,
**dadurch gekennzeichnet,**
**dass** das Drahtseil aus einer ungeraden Zahl von Litzen (14) mit jeweils einer ungeraden Zahl von Drähten (15) ausgebildet ist.

5. Sondenapplikator nach einem der Ansprüche 1 bis 4
**dadurch gekennzeichnet,**
**dass** die flexible Sondenhülle (6) im Querschnitt ringförmig ausgebildet ist und mindestens einer der elektrischen Leiter (7) eine isolierende Hülle (21) aufweist

6. Sondenapplikator nach einem der Ansprüche 1 bis 4,
**dadurch gekennzeichnet,**
**dass** die flexible Sondenhülle im Querschnitt innen eine sich mittig verengende Kontur aufweist in der die elektrischen Leiter beabstandet zueinander geführt werden.

7. Sondenapplikator nach einem der Ansprüche 1 bis 4,
**dadurch gekennzeichnet,**
**dass** die flexible Sondenhülle (6') eine Trennwand (22) aufweist, die das Lumen der Sondenhülle (6') in zwei voneinander getrennte Kammern (23, 24) unterteilt, in denen die elektrischen Leiter (7, 8) geführt werden.

8. Sondenapplikator nach einem der Ansprüche 1 bis 7,
**dadurch gekennzeichnet,**
**dass** die Sondenhülle (6, 6', 6") aus einem elektrisch isolierenden Kunststoff ausgebildet ist.

## Claims

1. Probe applicator (1) and electrosurgical probe (2) with a bendable distal probe end (5) having a probe tip with a bipolar electrode head (9) that is connected to two electrical conductors (7, 8) arranged in a probe casing (6, 6', 6"); wherein the probe applicator is designed for positioning the probe, with a shaft tube (3) in which the probe (2) is arranged, and a handpiece (4) with which it is possible to flex the probe end (5), wherein the shaft tube (3) is of rigid design and the shaft tube (3) and probe casing (6, 6', 6") are longitudinally displaceable relative to one another in the longitudinal direction, wherein the distal probe end (5) is pre-curved and is tensioned into an extended position when it is covered by the distal shaft tube end (17),
**characterized in that**,
in the region of the distal probe end (5), the first electrical conductor (7) has a directionally curved pre-tensioning so that, when the shaft tube end (17) is retracted, the free probe end (5) of the flexible probe (2) is pushed into its bent, pre-curved position and when the shaft tube (3) is advanced, the probe end (5) is tensioned into its extended position by the surrounding shaft tube end (17),
and **in that** the second electrical conductor (8) is designed as a flexible wire cable.

2. Probe applicator according to Claim 1,
**characterized in that**,
in the region of the curved pre-tensioning of the distal probe end (5), the first electrical conductor (7) is designed to be flat, with two opposing side faces (12, 13).

3. Probe applicator according to Claim 1 or 2,
**characterized in that**,
the flexible wire cable consists of a plurality of strands (14), each of which is composed of a plurality of wires (15).

4. Probe applicator according to Claim 3,
**characterized in that**,
the wire cable consists of an odd number of strands (14), each of which has an odd number of wires (15).

5. Probe applicator according to any of Claims 1 to 4
**characterized in that**,
the cross-section of the flexible probe casing (6) is ring-shaped and at least one of the electrical conductors (7) has an insulating cover (21).

6. Probe applicator according to any of Claims 1 to 4,
**characterized in that**,
internally the cross-section of the flexible probe casing has a narrowed contour in the middle in which the electrical conductors are arranged at a distance from one another.

7. Probe applicator according to any of Claims 1 to 4,
**characterized in that**,
the flexible probe casing (6') has a separator wall (22) that divides the lumen of the probe casing (6') into two separate chambers (23, 24) in which the electrical conductors (7, 8) are arranged.

8. Probe applicator according to any of Claims 1 to 7,
**characterized in that**,
the probe casing (6, 6', 6") is made of an electrically insulating plastic.

## Revendications

1. Applicateur de sonde (1) et sonde électrochirurgicale bipolaire (2) dotée d'une extrémité de sonde distale (5) pouvant être courbée, extrémité qui présente une pointe de sonde dotée d'une tête d'électrode bipolaire (9) qui est reliée à deux conducteurs électriques (7, 8) guidés dans une gaine de sonde (6, 6', 6") ;
sachant que l'applicateur de sonde est conçu pour positionner la sonde, avec une tige tubulaire (3) dans laquelle est guidée la sonde (2), et avec une poignée (4) au moyen de laquelle l'extrémité (5) de la sonde peut être courbée, sachant que la tige tubulaire (3) est réalisée rigide et que la tige tubulaire (3) et la gaine de sonde (6, 6', 6") sont longitudinalement coulissantes l'une par rapport à l'autre en direction longitudinale, sachant que l'extrémité distale (5) de la sonde est précambrée et est tendue dans une position étirée lorsqu'elle est couverte par l'extrémité distale (17) de la tige tubulaire,
**caractérisés en ce que** le premier conducteur électrique (7) présente, dans la région de l'extrémité distale (5) de la sonde, une précontrainte directionnellement cintrée par laquelle, lorsque l'extrémité (17) de la tige tubulaire est rétractée, l'extrémité (5) se trouvant à découvert de la sonde flexible (2) est poussée dans sa position courbée, précambrée, et, lorsque la tige tubulaire (3) est déployée, l'extrémité (5) de la sonde est tendue dans sa position étirée par l'extrémité (17) de la tige tubulaire qui l'entoure,
et **en ce que** le deuxième conducteur électrique (8) est réalisé sous la forme d'un câble métallique flexible.

2. Applicateur de sonde selon la revendication 1, **caractérisé en ce que** le premier conducteur électrique (7) est, dans la région de la précontrainte cintrée de l'extrémité distale (5) de la sonde, réalisé plat avec deux faces latérales (12, 13) mutuellement opposées.

3. Applicateur de sonde selon la revendication 1 ou 2, **caractérisé en ce que** le câble métallique flexible est constitué d'une pluralité de torons (14) comprenant chacun une pluralité de fils métalliques (15).

4. Applicateur de sonde selon la revendication 3, **caractérisé en ce que** le câble métallique est constitué d'un nombre impair de torons (14) comprenant chacun un nombre impair de fils métalliques (15).

5. Applicateur de sonde selon l'une des revendications 1 à 4, **caractérisé en ce que** la gaine de sonde flexible (6) est réalisée annulaire en coupe transversale et au moins un des conducteurs électriques (7) présente une gaine isolante (21).

6. Applicateur de sonde selon l'une des revendications 1 à 4, **caractérisé en ce que** la gaine de sonde flexible présente intérieurement en coupe transversale un contour se rétrécissant au milieu, dans lequel les conducteurs électriques sont guidés à distance entre eux.

7. Applicateur de sonde selon l'une des revendications 1 à 4, **caractérisé en ce que** gaine de sonde flexible (6') présente une paroi séparatrice (22) qui divise l'intérieur de la gaine de sonde (6') en deux chambres (23, 24) séparées l'une de l'autre, dans lesquelles sont guidés les conducteurs électriques (7, 8).

8. Applicateur de sonde selon l'une des revendications 1 à 7, **caractérisé en ce que** la gaine de sonde (6, 6', 6") est constituée d'une manière plastique électriquement isolante.
